# EUROPÄISCHE PATENTSCHRIFT

(11) **EP 1 058 657 B1**
(45) Veröffentlichungstag und Bekanntmachung des Hinweises auf die Patenterteilung: **28.05.2003**
(21) Anmeldenummer: 99910294.0
(22) Anmeldetag: 26.02.1999
(51) Int. Cl.: B65D 75/58, B65D 83/14

(54) **VERWENDUNG EINES BEHÄLTERS FÜR EINE MEDIZINISCHE FLÜSSIGKEIT IN EINEM ZERSTÄUBER**
USE OF A CONTAINER FOR A MEDICINAL LIQUID IN A SPRAYING DEVICE
UTILISATION D'UN RECIPIENT POUR LIQUIDE MEDICAL DANS UN DIFFUSEUR

(30) Priorität: 27.02.1998 DE 19808295
(43) Veröffentlichungstag der Anmeldung: 13.12.2000
(60) Teilanmeldung: 03002940.9
(73) Patentinhaber: Boehringer Ingelheim International GmbH, 55218 Ingelheim am Rhein (DE)
(72) Erfinder: KLADDERS, Heinrich, D-45468 Muelheim (DE); ZIERENBERG, Bernd, D-55411 Bingen (DE); HOCHRAINER, Dieter, D-55411 Bingen (DE); FREUND, Bernhard, D-55435 Gau-Algesheim (DE); EICHER, Joachim, D-44227 Dortmund (DE); GESER, Johannes, D-44227 Dortmund (DE); ESSING, Martin, D-44139 Dortmund (DE); REINECKE, Holger, D-44227 Dortmund (DE)
(74) Vertreter: Laudien, Dieter, Dr.
(86) Internationale Anmeldenummer: EP9901262
(87) Internationale Veröffentlichungsnummer: WO99043571

(56) Entgegenhaltungen:
- EP-A- 0 585 908
- EP-A- 0 654 419
- EP-A- 0 763 482
- WO-A-95/15895
- US-A- 4 440 316

## Beschreibung

Die Erfindung betrifft einen Behälter für eine medizinische Flüssigkeit, der gas- und flüssigkeitsdicht ist.

Die Erfindung bezweckt, die Herstellung eines derartigen für einmaligen Gebrauch bestimmten Behälters wirtschaftlicher zu gestalten, ohne seine Brauchbarkeit zu beeinträchtigen, sowie seine Handhabung zu vereinfachen.

In EP - 0 182 094 A2 ist eine flaschenförmige Verpackung angegeben, die aus einem formsteifen Außenbehälter und einem darin befindlichen Innenbehälter besteht, der als leicht verformbarer Beutel für das Füllgut ausgebildet ist. Der Vorformling wird durch Koextrusion zweier koaxialer schlauchförmiger Abschnitte hergestellt. Die beiden Abschnitte bestehen aus zwei thermoplastischen Kunststoffen, die keine Verbindung miteinander eingehen. Der Vorformling wird in einer Blasform aufgeweitet. Am flachen Boden des Innenbehälters ist eine Schweißnaht vorhanden. Der am Außenbehälter angeformte flache Boden enthält eine schlitzförmige Öffnung. Im Bereich der Entnahmeöffnung sind Außenbehälter und Innenbehälter formschlüssig miteinander verbunden. Diese Verpackung wird im wesentlichen in einem Arbeitsgang hergestellt.

Das Füllgut wird mittels einer in der Entnahmeöffnung angebrachten Pumpe entnommen, wobei sich der Innenbehälter unter Abnahme seines Volumens verformt. Durch den offenen Schlitz im flachen Boden des Außenbehälters tritt Luft in den Raum zwischen Außenbehälter und verformtem Innenbehälter ein, wodurch die Entstehung eines Unterdruckes in diesem Zwischenraum verbindert wird. Der Innenbehälter hat - außer im Bereich der EntnahmeÖffnung - keinen festen Kontakt zum Außenbehälter. Die Verpackung kann mit einem fast bis zum flachen Boden reichenden Tauchrohr versehen sein, das den Innenbehälter in gestreckter Lage hält. Diese Verpackung ist nur bei bestimmter räumlicher Lage einwandfrei benutzbar und vollständig entleerbar.

In EP - 0 620 165 A1 wird ein Schlauchbeutel aus Verbundfolie beschrieben. Die Verbundfolie besteht zumindest aus einer außenliegenden Kunststoff-Folie und einer innenliegenden Metallfolie. Der Schlauchbeutel ist an beiden Enden sackartig verschlossen. Der Schlauchbeutel ist mit einer Sollbruchstelle versehen, mittels der er sich an dieser Stelle zuverlässig öffnen läßt. Ein derartiger Scblauchbeutel dient zum Lagern einer aushärtbaren Masse, die mittels eines Auspressgerätes aus dem Schlauchbeutel ausgepreßt wird.

EP - 0 068 653 A1 beschreibt einen zur einmaligen Benutzung vorgesehenen aus einer Folie hergestellten flexiblen und kollabierbaren Behälter, der in einer mehrfach verwendbaren Saugflasche eingesetzt wird. Das eine Ende des Behälters ist offen, das andere Ende ist mittels einer Schweißnaht verschlossen und mit einer Lasche versehen. Die Lasche ist in einem am Boden der Saugflasche vorhandenen Spalt eingeklemmt. Dadurch wird der beutelartige Behälter in der Saugflasche ständig in gestreckter Lage gehalten.

Die EP 0 322 980 offenbart einen Beutel mit einem aufgeklebten Entnahmestutzen in Form eines hohlen Rohres zum Herausnehmen des Inhalts des Beutels. Im Inneren des Beutels, gegenüber dem Entnahmestutzen ist ein Sporn so ausgebildet, dass wenn die Seite mit dem Sporn auf die Seite mit dem Entnahmestutzen gedrückt wird, der Sporn den Bereich des Beutels, der von außen durch den rohrförmigen Entnahmestutzen geschützt wird, durchbohrt wird und dadurch den Beutel öffnet.

Aufgabe der vorliegenden Erfindung ist, eine Kartusche für einen treibgasfreien Inhalator zu finden, wobei die Kartusche eine medizinische Flüssigkeit enthält, die Kartusche gas- und flüssigkeitsdicht ist, deren Füllvolumen an den vorgesehenen Verwendungszweck anpaßbar ist, und die bei geringem Unterdruck in vorgegebener Weise plastisch und irreversibel kollabiert und annähernd vollständig entleerbar ist.

Diese Aufgabe wird erfindungsgemäß gelöst durch die Verwendung nach Anspruch 1.

In einer weiteren Ausführungsform kann der kollabierbare Folienbeutel bereits bei einem Differenzdruck unterhalb 150 hPa (150 mbar) oder bevorzugt unterhalb 80 hPa (80 mbar) durch den Außendruck verformt werden und kollabieren.

Der Folienbeutel kann an beiden Enden durch eine Schweißnaht verschlossen sein. In diesem Fall ist der formstabile Flansch an der Seite des Folienbeutels, bevorzugt in der Nähe eines Endes des Folienbeutels, dicht angeschweißt. Der Folienbeutel kann jedoch auch an einem Ende durch eine Schweißnaht und am anderen Ende durch den formstabilen Flansch dicht verschlossen sein. In diesem Fall ist das eine Ende des Folienbeutels mit dem formstabilen Flansch, bevorzugt auf dessen Umfang, verschweißt.

Der formstabile Flansch kann verschieden geformt sein. Falls er am Ende des Folienbeutels als dessen Verschluß angebracht ist, kann er rotationssymmetrisch geformt und an die Größe des Endes des Folienbeutels angepaßt sein. Der formstabile Flansch kann mit einem Führungskanal versehen sein, in den der Entnahmestutzen eingeführt wird und in dem sich der Entnahmestutzen bei aufgestecktem Behälter befindet.

Es kann zweckmäßig sein, den Führungskanal mit einer Preßpassung zu versehen, die den Entnahmestutzen umschließt. Die Preßpassung kann ein Abschnitt des Führungskanals sein, der aus einer glatten Innenwand besteht mit einem Innendurchmesser, der von dem Außendurchmesser des Entnahmestutzens nur wenig abweicht. In einer weiteren Ausführungsform können in einem Abschnitt des Führungskanals auf dessen Innenwand mehrere Ausbuchtungen vorhanden sein. Die Ausbuchtungen können zum Beispiel drei in axialer Richtung verlaufende symmetrisch angeordnete und länglich geformte Ausbuchtungen sein. Weiter können mehrere, in einem axialen Abstand voneinander angeordnete und in azimutaler Richtung verlaufende Ausbuchtungen vorgesehen sein, die zum Beispiel zwei Ringe bilden, oder die aus mehreren Ringabschnitten bestehen. Ferner können die Ausbuchtungen wendelförmig verlaufen; sie können aus mehreren auf der Innenwand des Führungskanals verteilten Wendelabschuitten oder aus einem Wendelabschnitt bestehen, dessen Länge größer ist als der Umfang des Führungskanals. Eine derartige Preßpassung ermöglicht das Aufstecken des Behälters auf den Entnahmestutzen sowie einen hinreichend festen Sitz des formstabilen Flansches auf dem Entnahmestutzen. Ferner kann der Behälter nach seiner Entleerung von dem Entnahmestutzen abgezogen werden, ohne den Entnahmestutzen zu beschädigen.

Der formstabile Flansch besteht aus Gummi, Metall oder Kunststoff, bevorzugt aus einem thermoplastischen Kunststoff. Es kann zweckmäßig sein, den formstabilen Flansch aus demselben Kunststoff herzustellen, aus dem der Folienbeutel oder die Innenseite des Folienbeutels besteht.

Die Schweißnaht an einem oder beiden Enden des Folienbeutels kann U-, V- oder T-förmig ausgebildet sein; sie verläuft im wesentlichen quer zur Beutelachse. Sie kann sich teilweise in Richtung der Beutelachse erstrecken, wodurch beim Entnehmen von Flüssigkeit die definierte Verformung des Folienbeutels begünstigt wird.

Innerhalb oder an einem der Enden des Führungskanals kann eine Dichtstelle vorgesehen sein. Die Dichtstelle kann aus einem Ring bestehen, der in einer auf der Innenwand des Führungskanals angebrachten Nut liegt. Der Querschnitt des Ringes kann O-förmig oder im wesentlichen rechteckig sein. Der Ring ist gegebenenfalls mit einer Dichtlippe versehen. Der Ring kann aus einem Elastomer, aus einem thermoplastischen Elastomer oder aus Gummi bestehen. Die Dichtstelle verschließt den Füllraum des auf den Entnahmestutzen aufgesteckten Behälters gegen die Umgebungsluft gas- und flüssigkeitsdicht. Sie erlaubt das Abziehen des entleerten Behälters von dem Entnahmestutzen. Die Dichtstelle ist erforderlich, falls die Dichtwirkung der Preßpassung nicht ausreicht.

Die Entnahmestelle ist bevorzugt als Einstichstelle ausgebildet. An der Einstichstelle kann eine durchstechbare Membran vorgesehen sein, die beim Aufstecken des Behälters auf den Entnahmestutzen durchstochen wird. Die Membran ist bevorzugt zwischen der Dichtstelle und dem Flüssigkeitsraum im Folienbeutel angeordnet. Die durchstechbare Membran kann an einem der Enden oder innerhalb des Führungskanals angebracht sein. Sie ist bevorzugt unmittelbar an dem Ende des Führungskanals oder in der Nähe dieses Endes angebracht, das dem Flüssigkeitsraum zugewandt ist. Sie kann ein Teil des formstabilen Flansches oder ein Teil des Folienbeutels sein. Falls sie ein Teil des formstabilen Flansches ist, kann sie gleichzeitig mit dem formstabilen Flansch hergestellt werden. Sie kann aus demselben Kunststoff wie der formstabile Flansch bestehen. Die durchstechbare Membran wirkt als originaler Verschluß für den Füllraum des Folienbeutels.

In einer weiteren Ausführungsform kann die Entnahmestelle mit einer Siegelfolie versiegelt sein, die vor dem Aufstecken des Behälters auf den Entnahmestutzen abgezogen wird, oder die beim Aufstecken des Behälters auf den Entnahmestutzen durchstochen wird.

Der formstabile Flansch kann einteilig oder mehrteilig sein. Der mehrteilige Flansch kann bevorzugt zweiteilig sein. Das außen liegende Teil des Flansches ist mit dem Folienbeutel dicht verbunden. Das äußere Teil enthält eine Öffnung, die mit dem inneren Teil dicht verschlossen ist. Beide Teile können mittels eines Gewindes miteinander verschraubt werden, oder mittels einer Schnappverbindung oder durch Ultraschallschweißen miteinander verbunden werden. Der einteilige Flansch ist analog zum zweiteiligen Flansch ausgebildet, er enthält jedoch keine Verbindungselemente.

Der formstabile Flansch kann gleichzeitig mit Preßpassung, Nut für die Dichtstelle und durchstechbarer Membran hergestellt werden.

Der Folienbeutel kann aus einem Schlauch bestehen, der keine in axialer Richtung des Folienbeutels verlaufende Schweißnaht aufweist. Ferner kann er aus einer Folie hergestellt werden und eine oder zwei in Längsrichtung verlaufende Schweißnähte haben. Er kann als Flachbeutel oder als Beutel mit Seitenfalten ausgebildet sein. Ein Beutel mit einer in Längsrichtung verlaufenden Schweißnaht wird bevorzugt.

Die Schweißnähte am Folienbeutel können von 0,7 mm bis 3 mm breit sein; ihre Breite wird entsprechend den an Dichtheit und Haltbarkeit der Naht gestellten Forderungen gewählt. Breite Längsnähte am Folienbeutel können nach dem Verschweißen umgebogen werden, damit sie außen am Folienbeutel etwa anliegen und der Folienbeutel nur wenig breiter ist als seine Breite im unverschweißten Teil zwischen den Schweißnähten.

Der Folienbeutel kann aus einer Folie aus Metall oder Metall-Legierung - bevorzugt aus Aluminium, Gold oder Kupfer - oder aus Kunststoff - bevorzugt einem Thermoplasten - bestehen. In einer anderen Ausführungsform kann der Folienbeutel aus einer Verbundfolie aus Kunststoff und Metall bestehen. Die Verbundfolie besteht bevorzugt aus zwei oder drei miteinander verbundenen Folien. Weiter kann der Folienbeutel aus einer Kunststoff-Folie bestehen, auf die eine Schicht aus Metall, Glas oder Keramik, zum Beispiel durch Aufdampfen, aufgebracht ist. Die Folien aus Kunststoff oder aus Metall sind einige Mikrometer dick. Die Dicke der Aufdampfschichten aus Metall, Glas oder Keramik liegt im Submikrometerbereich.

Die Verbundfolie aus zwei Folien kann aus einer Metallfolie und einer Kunststoff-Folie bestehen, die miteinander verbunden sind. Die Metallfolie bildet die Innenseite oder die Außenseite der Verbundfolie. In einer anderen Ausführungsform besteht die Verbundfolie aus zwei unterschiedlichen Kunststoffen.

Die Verbundfolie aus drei Folien besteht bevorzugt aus zwei Kunststoff-Folien, zwischen denen eine Folie aus Metall liegt. Alle drei Folien sind miteinander verbunden. An Stelle der Metallfolie kann eine Schicht aus Glas oder Keramik vorhanden sein, zum Beispiel aus Siliziumoxid (SiOₓ), die auf eine Kunststoff-Folie aufgedampft ist.

In einer weiteren Ausführungsform besteht die Innenfolie der Verbundfolie aus einem Copolymer, zum Beispiel einem Polyethylen-Copolymer aus Ethylen-Acrylsäure. Für die äußere Kunststoff-Folie der verbundfolie wird bevorzugt ein Kunststoff verwendet, zum Beispiel Polyethylen-terephthalat, dessen Schmelztemperatur größer ist als die Schmelztemperatur des Kunststoffs der Innenfolie. Hierdurch wird beim Herstellen des Folienbeutels das nahtweise Verschweißen des Kunststoffs der Innenfolie erleichtert.

Bei der Verbundfolie kann zwischen zwei Folien gegebenenfalls eine Haftvermittlerschicht vorhanden sein.

Der Folienbeutel kann aus einer Kunststoff-Folie mit einer Dicke von 20 µm bis 100 µm bestehen. Weiter kann er aus einer Verbundfolie mit einer Innenfolie aus Kunststoff mit einer Dicke von 20 µm bis 100 µm und einer Außenfolie aus Metall mit einer Dicke von 8 µm bis 20 µm bestehen, Ferner kann er aus einer Verbundfolie mit einer Innenfolie aus Kunststoff mit einer Dicke von 20 µm bis 100 µm, einer Mittelfolie aus Metall mit einer Dicke von 8 µm bis 20 µm und einer Außenfolie aus Kunststoff mit einer Dicke von 10 µm bis 40 µm bestehen.

Die Schweißnähte am Folienbeutel sowie die Schweißstelle zwischen Folienbeutel und formstabilem Flansch werden nach bekannten Verfahren hergestellt, wie thermisches Schweißen, Ultraschallschweißen oder Induktions-Schweißen bei Verbundfolien mit metallener Schicht, wobei die Schweißstellen im erhitzten Zustand bevorzugt zusammengedrückt werden. Derartige Verfahren sind beispielsweise in EP - 0 111 131 und EP - 0 130 239 angegeben.

Ein formstabiler Flansch aus Gummi oder aus Metall kann mit dem Folienbeutel durch Kleben oder gegebenenfalls durch Vulkanisieren verbunden werden.

Der Behälter kann sich in einer formstabilen Hülse aus Metall oder aus Kunststoff befinden, deren eines Ende mit dem formstabilen Flansch lösbar oder unlösbar verbunden ist, und deren anderes Ende gegebenenfalls mit einem Boden verschlossen ist. Die Hülse kann im wesentlichen rundum geschlossen sein. Sie enthält jedoch mindestens eine Öffnung, oder an der Verbindungsstelle mit dem Flansch befindet sich ein Spalt. weiter kann die Hülse als formstabiler Korb mit vielen Öffnungen ausgebildet sein. Der Behälter kann sich an Stelle der Hülse in einem formstabilen U-förmigen Bügel befinden, wobei das Ende jedes Schenkels des Bügels an dem formstabilen Flansch befestigt ist und die Schenkel länger sind als der Folienbeutel.

Der in einer Hülse befindliche Behälter ist nur am formstabilen Flansch mit der Hülse verbunden. Das mit einer Schweißnaht verschlossene Ende oder die beiden mit einer Schweißnaht verschlossenen Enden des Folienbeutels sind mit der Hülse nicht verbunden.

Beim Übergang von Flüssigkeit aus dem Behälter in den Entnahmestutzen legt sich der Folienbeutel durch die Wirkung des Außendruckes flach zusammen. Durch die Öffnung in der Hülse oder durch den Spalt zwischen Hülse und formstabilem Flansch tritt Luft in den Raum zwischen der Hülse und dem Folienbeutel ein und führt den Druckausgleich herbei. Damit ist im Folienbeutel kein Ventil erforderlich, und die Flüssigkeit im Folienbeutel kommt nicht mit Luft in Berührung.

Der Folienbeutel ist diffusionsdicht für die medizinische Flüssigkeit und ihre Bestandteile sowie für Gase. Das Material für den Folienbeutel und gegebenenfalls der Aufbau der Verbundfolie werden dementsprechend gewählt. Diffusionsdicht im Sinne der vorliegenden Erfindung bedeutet einen Verlust an Flüssigkeit (gemessen mit Ethanol bei Raumtemperatur) des Behälters durch Diffusion von weniger als 0,6 mg pro Tag, bevorzugt weniger als 0,4 mg pro Tag, besonders bevorzugt weniger als 0,2 mg pro Tag, insbesondere weniger als 0,1 mg pro Tag.

Die Innenfolie oder die Innenseite des Folienbeutels steht mit der eingefüllten Flüssigkeit in Kontakt. Für diese Folie wird ein Material gewählt, das durch die Flüssigkeit nicht angegriffen wird und durch das die Flüssigkeit nicht beeinträchtigt wird. Diese Folie wird bevorzugt als schweißfähige Folie ausgelegt.

Eine der Folien oder eine zum Beispiel aufgedampfte Schicht ist die Diffusionssperre, die die Diffusion der Flüssigkeit oder ihrer Bestandteile sowie die Diffusion von Gasen aus dem oder in den Folienbeutel verhindert. Es kann zweckmäßig sein, die Diffusionssperre gegen mechanische Beschädigung und gegen ein Zerreißen der Diffusionssperre beim Biegen der Folie durch eine weitere auf die Diffusionssperre aufgebrachte Kunststoff-Folie zu schützen, damit die Diffusion von Flüssigkeit oder von Gasen dauerhaft verhindert bleibt.

Da der Folienbeutel diffusionsdicht gegen Gase ist, kann der durch die Plüssigkeitsentnahme entstehende Unterdruck im Folienbeutel nicht durch eindiffundierendes Gas ausgeglichen werden, und der Folienbeutel kollabiert zuverlässig auch bei sehr langsamer Entnahme von Flüssigkeit aus dem Behälter. Die Flüssigkeit kann auch in vielen Teilmengen, zum Beispiel 200 Dosierungen, verteilt über eine längere Zeit, zum Beispiel drei Monate, aus dem Folienbeutel entnommen werden.

Der in einer im wesentlichen geschlossenen Hülse befindliche Behälter ist von außen unzugänglich und kann bei der Lagerung und beim Aufstecken auf den Entnahmestutzen nicht beschädigt werden. Die im wesentlichen geschlossene Hülse oder die als Korb mit vielen Öffnungen versehene Hülse oder der formstabile Bügel erleichtern die Lagerung des Behälters mit dem dünnwandigen Folienbeutel und seine Handhabung beim Aufstecken auf den Entnahmestutzen und beim Abziehen des leeren Behälters von dem Entnahmestutzen.

Der Entnahmestutzen ist erfindungsgemäß der Hohlkolben eines Zerstäubers für medizinische Flüssigkeiten. Ein derartiger Zerstäuber ist in DE - 195 36 902.5 und in WO - 97/12687 (speziell in den dortigen Figuren 6a und 6b) beschrieben. Der Hohlkolben dieses Zerstäubers ist als Entnahmestutzen für die in dem erfindungsgemäßen Behälter enthaltene medizinische Flüssigkeit ausgebildet. Der Behälter wird auf den bevorzugt in der Achse des Zerstäubers angebrachten Hohlkolben aufgesteckt, wobei das Ende des Hohlkolbens in die Entnahmestelle sticht und damit in die medizinische Flüssigkeit eintaucht. Die Dichtstelle im formstabilen Flansch schließt den Innenraum des Behälters gegen die Außenwand des Hohlkolbens dicht ab. Die Preßpassung kann den Behälter auf dem Hohlkolben mechanisch festhalten.

Es kann zweckmäßig sein, anstelle der oder zusätzlich zur Preßpassung (kraftschlüssigen Verbindung) zwischen Behälter und Entnahmestutzen eine lösbare, formschlüssige Verbindung zwischen dem formstabilen Flansch des Behälters und dem Entnahmegerät, zum Beispiel einem Zerstäuber, vorzusehen. Eine derartige Verbindung kann als steckbare Schnappverbindung aus mehreren Schnapphaken bestehen, die in einem Anschlußstück im Entnahmegerät angebracht sind. Beim Einstecken des Behälters in das Entnahmegerät greifen die Schnapphaken in eine Aussparung im Flansch, zum Beispiel in eine umlaufende Rille oder hinter eine Kante des formstabilen Flansches, ein. Die Schnappnasen sind bevorzugt rund geformt oder in beiden Bewegungsrichtungen des Behälters abgeschrägt, um einen leeren Behälter mit mäßig großem Kraftaufwand entnehmen zu können und einen vollen Behälter in das Entnahmegerät einstecken zu können.

Der erfindungsgemäße Behälter eignet sich besonders als auswechselbare Kartusche für inhalierbare Arzneimittel-Lösungen in treibgasfreien Zerstäubern. Das Füllvolumen des Behälters kann von 0,5 Milliliter bis 5 Milliliter, bevorzugt von 1 ml bis 4 ml und besonders bevorzugt von 1 ml bis 3 ml oder von 2 ml bis 4 ml betragen. Diese Lösungen werden portionsweise mit einer Dosis jeweils von 10 Mikroliter bis 50 Mikroliter, bevorzugt von 15 µl bis 20 µl entnommen.

Der Hülsendurchmesser kann von 10 mm bis 30 mm betragen, bevorzugt von 12 mm bis 17 mm. Die Länge des Behälters einschließlich des aus der Hülse hervorstehenden Teils des formstabilen Flansches kann von 20 mm bis 60 mm, bevorzugt von 30 mm bis 50 mm betragen.

Der erfindungsgemäße Behälter dient als Primärpackmittel zur Aufnahme einer medizinischen Flüssigkeit, zum Beispiel ein in einem Lösemittel gelöstes Arzneimittel. Als Lösemittel sind beispielsweise Wasser, Ethanol oder deren Mischungen geeignet. Als Arzneimittel werden beispielsweise Berotec (Fenoterol-Hydrobromid;1-(3,5-dihydroxy-phenyl)-2-[[1-(4-hydroxy-benzyl)-ethyl]-amino]-ethanol-hydrobromid), Atrovent (Ipratropiumbromid), Berodual (Kombination aus Fenoterol-Hydrobromid und Ipratropiumbromid), Salbutamol (oder Albuterol), Combivent, Oxivent (Oxitropiumbromid), Ba 679 (Tiotropiumbromid), BEA 2108 (Di-(2-thienyl)-glykolsäuretropenolester), Flunisolid, Budesonid, Beclomethason und andere verwendet.

In WO - 98/27959 werden stabilisierte wässrige Arzneimittel-Zubereitungen zum Herstellen von treibgasfreien Aerosolen für die Inhalation beschrieben. Auf die dort beanspruchten und in den Beispielen angegebenen Formulierungen wird Bezug genommen.

Geeignete Arzneimittel-Zubereitungen in ethanolischer Lösung sind beispielsweise in WO - 97/01329 angegeben, insbesondere wird auf die dort genannten Wirkstoffe (siehe dort die Seiten 2 und 3) sowie die dort beanspruchten stabilisierten Formulierungen Bezug genommen.

Die erfindungsgemäße Verwendung hat folgende Vorteile:
- Er ist wirtschaftlich herstellbar, für einmaligen Gebrauch geeignet und benötigt einen nur geringen Materialeinsatz.
- Er ist steril herstellbar, steril befüllbar und steril versiegelbar.
- Er ist für zum Inhalieren vorgesehene Arzneimittel verwendbar, die als Lösungen in Ethanol, Ethanol/Wasser oder Wasser vorliegen.
- Die Flüssigkeit wird steril entnommen; dabei wird keine Luft in den Behälter eingesaugt. Die Flüssigkeit kommt mit Luft, Sauerstoff oder Kohlendioxid nicht in Berührung.
- Der erfindungsgemäße Behälter ermöglicht die gasblasenfreie Entnahme der medizinischen Flüssigkeit.
- Er ist dicht gegen die Diffusion von Flüssigkeiten und Gasen.
- Er ist im befüllten Zustand je nach Arzneimittel über mehrere Jahre lagerfähig und erfüllt die Forderungen sämtlicher amtlicher Arzneibücher (Phannakopöen).
- Er ist bereits bei einem geringen Unterdruck leicht verformbar. Im kollabierten Zustand bleibt er flach und annähernd gestreckt, er behält seine Ausgangslänge nach dem Entleeren annähernd bei.
- Er benötigt kein Ventil für den Druckausgleich nach dem Entnehmen eines Teils der Flüssigkeit.
- Der Behälter läßt sich weitgehend entleeren, auch bei wechselnder Lage und bei Überkopflage.
- Der Folienbeutel ist nur mit dem formstabilen Flansch verbunden. Er ist nicht an der gegebenenfalls vorhandenen Hülse befestigt.
- Sein Füllvolumen kann durch Ändern der Länge und/oder des Durchmessers des Folienbeutels leicht auf einen vorgegebenen Wert innerhalb eines gewissen Bereiches eingestellt werden.
- Er kann vor dem Verschließen befüllt werden, bevor die einzige oder die zweite Schweißnaht angebracht wird; ein besonderer Verschluß ist nicht erforderlich.
- Der Bebälter kann ohne oder mit einer Hülse verwendet werden.
- Der in einer Hülse befindliche Behälter ist vor außeren Beschädigungen geschützt.
- Die Flüssigkeit im Folienbeutel ist durch einen undurchsichtigen Folienbeutel oder durch eine rundum geschlossene undurchsichtige Hülse gegen Lichteinwirkung geschützt.
- Er läßt sich auf einfache Weise und ohne Drehbewegung in ein Entnahmegerät einstecken und entnehmen.

Der zur Erfindung gehörende Behälter wird an Hand der beispielhaften Figuren weiter erläutert.

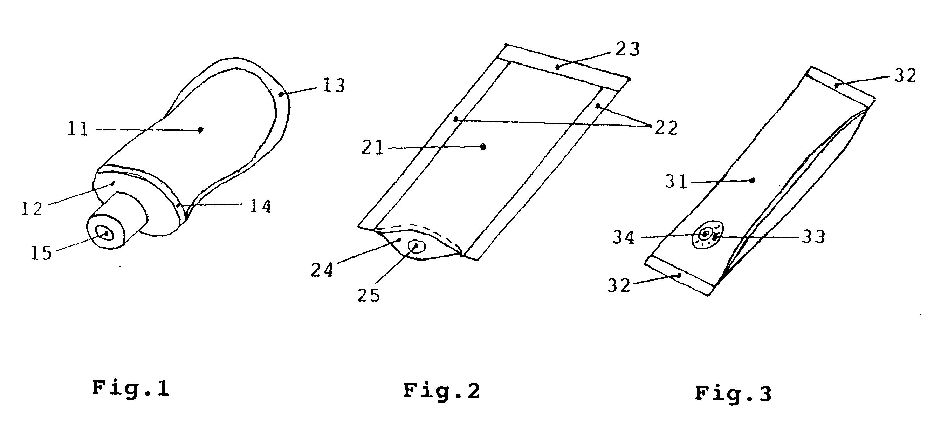

Die Figuren 1 bis 3 zeigen in Schrägansicht verschiedene Ausführungsformen des an beiden Enden verschlossenen Folienbeutels.

Figur 1 zeigt einen Schlauchbeutel (11) mit zylindrischem formstabilen Flansch (12) und U-förmiger Quernaht (13), die das eine Ende des Schlauchbeutels verschließt und sich teilweise in Längsrichtung des Schlauchbeutels erstreckt. Der Rand (14) des Flansches ist mit dem anderen Ende des Schlauchbeutels verbunden. In der Achse des Flansches befindet sich ein Loch (15), in das ein Entnahmestutzen eingeführt werden kann.

Figur 2 zeigt einen Siegelrandbeutel (21), der aus zwei aufeinanderliegenden Folien besteht. Er hat zwei in Längsrichtung des Beutels verlaufende Schweißnähte (22) und an seinem einen Ende eine in Querrichtung verlaufende Schweißnaht (23). Das andere Ende ist mit einem fischartig geformten formstabilen Flansch (24) verbunden. In der Mitte des Flansches befindet sich ein Loch (25), in das ein Entnahmestutzen eingeführt werden kann.

Figur 3 zeigt einen Seitenfalten-Beutel (31) mit Falten an beiden Längsseiten, der an beiden Enden mit je einer guer verlaufenden Schweißnaht (32) verschlossen ist. Auf einer Flachseite des Beutels ist der formstabile Flansch (33) aufgeschweißt. In das Loch (34) des Flansches kann ein Entnahmestutzen eingeführt werden.

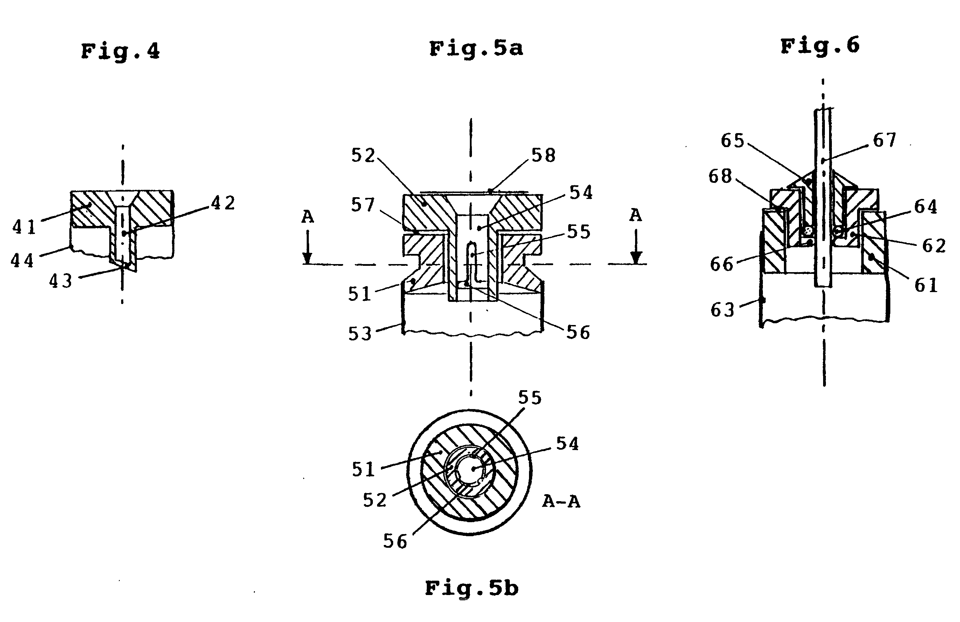

In den Figuren 4 bis 7 sind verschiedene Ausführungsformen des formstabilen Flansches im Schnitt dargestellt.

Figur 4 zeigt einen Längsschnitt durch einen einstückigen Flansch (41) mit einem als Preßpassung ausgebildeten zylindrischen Führungskanal (42) für einen zylindrischen Entnahmestutzen. Das äußere Ende des Führungskanals ist angeschrägt, das andere Ende ist mit einer zur Flanschachse geneigt angebrachten Membran (43) verschlossen. Dieser Flansch wird in einem Arbeitsgang gefertigt. Der Rand des Flansches ist mit einem Folienbeutel (44) verbunden.

Figur 5 a zeigt einen mehrteiligen zylindrischen Flansch im Längsschnitt und Figur 5 b im Querschnitt an der in Figur 5 a mit A-A gekennzeichneten Linie. Das Unterteil (51) ist mit einem Folienbeutel (53) verbunden. Das Oberteil (52) steckt in einer Öffnung des Unterteils. Das Oberteil ist mit einem Führungskanal (54) versehen, in dem drei in Achsenrichtung des Flansches verlaufende längliche Wülste (55) als Preßpassung für einen Entnahmestutzen sowie eine ringförmigen Wulst (56) als Dichtung vorhanden ist. Unterteil (51) und Oberteil (52) sind an ihrer Berührungsfläche (57) miteinander verschweißt. Die Eintrittsöffnung mit Einführungsschräge des Führungskanals ist mit einer Siegelfolie (58) versiegelt.

In Figur 6 ist ein mehrteiliger zylindrischer formstabiler Flansch dargestellt. Das Unterteil (61) ist mit einem Folienbeutel (63) verbunden. Das Oberteil (62) ragt in das ringförmige Unterteil hinein. Im Oberteil befindet sich ein O-Ring (64) als Dichtung, der durch eine eingepreßte Stopfbuchse (65) gehalten wird. Die Öffnung in der Stopfbuchse dient als Führungskanal für einen Entnahmestutzen. Auf der Innenseite der Dichtung befindet sich am Oberteil eine umlaufende Wulst (66), die als Preßpassung ausgebildet ist. In den Führungskanal ist ein Entnahmestutzen (67) eingeschoben. Der Behälter wird mittels der wulstförmigen Preßpassung (66) auf dem Entnahmestutzen gehalten. Unterteil (61) und Oberteil (62) sind an ihrer Berührungsfläche (68) miteinander verschweißt.

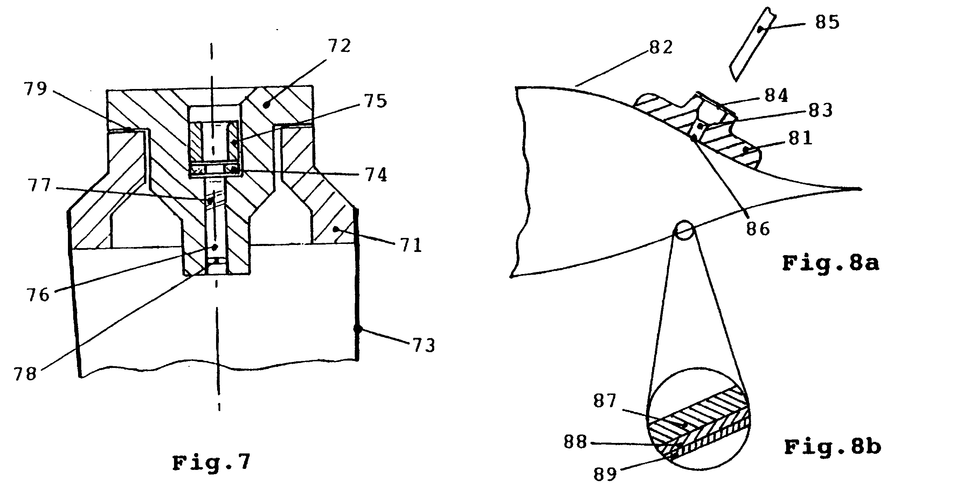

In Figur 7 ist ein weiterer mehrteiliger Flansch dargestellt. Das Unterteil (71) ist mit einem Folienbeutel (73) verbunden. Das Oberteil (72) befindet sich in dem mit einer Schulter versehenen ringförmigen Unterteil. Das Oberteil enthält einen flachen Ring (74) als Dichtung, der durch eine eingepreßte Stopfbuche (75) gehalten wird. Unterhalb der Flachdichtung befindet sich der Führungskanal (76) für einen Entnahmestutzen. Die Preßpassung besteht aus zwei gewindeartig umlaufenden Wülsten (77) innerhalb des Führungskanals. In der Nähe des unteren Endes des Führungskanals ist eine Membran (78) senkrecht zur Flanschachse angebracht, die beim Aufstecken des Behälters auf den Entnahmestutzen durchstochen wird. Die Membran wird mit dem Oberteil in einem Arbeitsgang hergestellt. Unterteil und Oberteil sind an ihrer Berührungsfläche (79) miteinander verbunden.

In Figur 8 a ist ein einstückiger formstabiler Flansch (81) im Querschnitt dargestellt, der auf der Seite eines Folieribeutels (82) angebracht ist. Der Flansch enthält eine Öffnung (83), die als Preßpassung für einen Entnahmestutzen dient. Dieser Flansch ist auf seiner Außenseite mit einer Siegelfolie (84) versiegelt. Beim Aufstecken des Behälters auf einen Entnahmestutzen (85) mit angeschrägtem Ende wird der Folienbeutel am inneren Ende (86) des Führungskanals durchstcchen.

Figur 8 b zeigt einen Querschnitt durch eine handelsübliche Laminat-Folie mit drei Schichten, aus der der Folienbeutel besteht. Die Innenfolie (87) besteht aus Polyethylen (40 µm dick), die Mittelfolie (88) ist die Diffusionssperre aus Aluminium (12 µm dick), und die Außenfolie (89) besteht aus Polyethylen-terephthalat (12 µm dick).

In den Figuren 9 bis 11 sind verschiedene Ausführungen der Schweißnaht, mit der der Folienbeutel an mindestens einem Ende verschlossen ist, in Seitenansicht und im Querschnitt (Figuren 9 b und 10 b) oder in der Aufsicht auf das Beutelende (Figur 11 b) dargestellt.

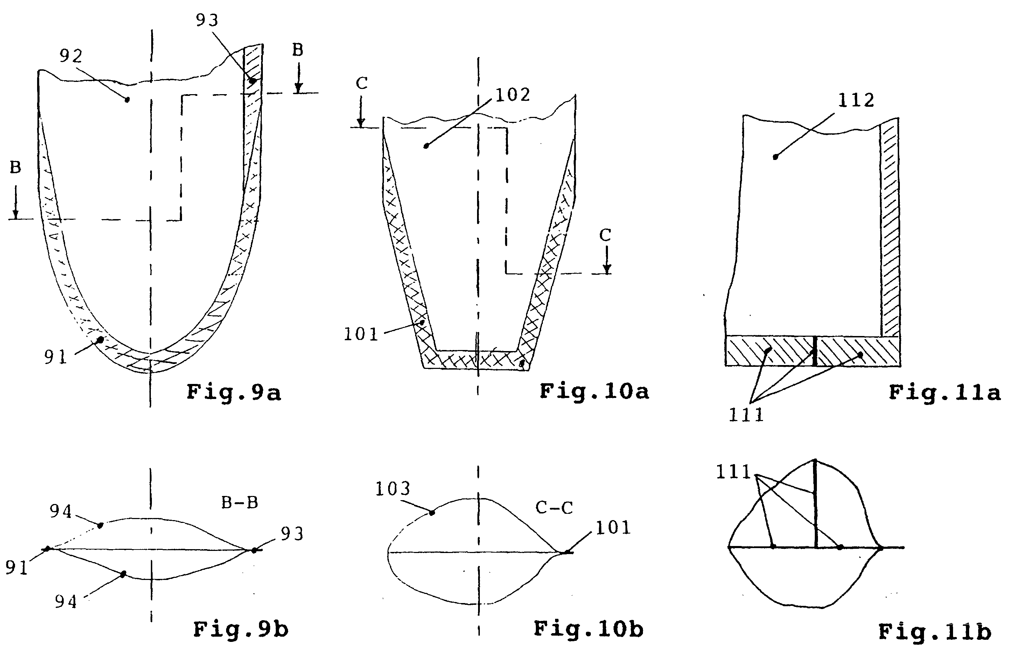

Figur 9 a zeigt eine U-förmige Schweißnaht (91), die sich teilweise in Längsrichtung des Folienbeutels (92) erstreckt und auf einer Seite in eine in Längsrichtung verlaufende Schweißnaht (93) des Folienbeutels übergeht. Figur 9 b zeigt einen Querschnitt durch den Folienbeutel an der in Figur 9a mit B-B gekennzeichneten Linie. In den Schweißnähten ist die Innenschicht der gefalteten Mehrschicht-Folie (94) verschweißt.

Figur 10 a zeigt eine V-förmige Schweißnaht (101), die sich teilweise in Längsrichtung des Folienbeutels erstreckt. In diesem Fall besteht der Folienbeutel aus einer Schlauchfolie ohne Längsnaht. Figur 10 b zeigt einen Querschnitt durch den Folienbeutel an der in Figur 10 a mit C-C gekennzeichneten Linie. In der Schweißnaht ist die gefaltete Einschicht-Folie (103) verschweißt.

Figur 11 a zeigt eine T-förmige Schweißnaht (111) in Seitenansicht und Figur 11 b in der Aufsicht auf das verschweißte Ende des Folienbeutels (112). Die drei Schenkel der T-Naht sind insgesamt so lang wie der flach zusammengelegte Folienbeutel außerhalb der T-Naht breit ist.

Der mit einer U-, v- oder T-Naht verschweißte Folienbeutel ist im Bereich der Quernaht nicht größer als der Durchmesser der Hülse, in die der Folienbeutel gegebenenfalls eingeschoben wird.

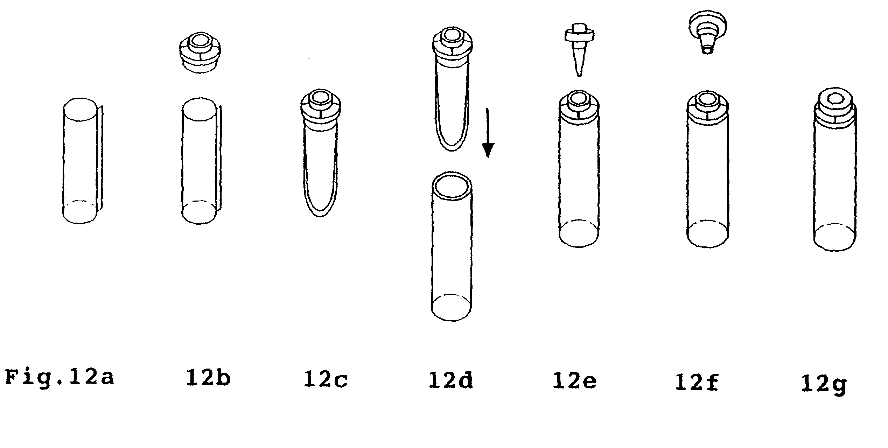

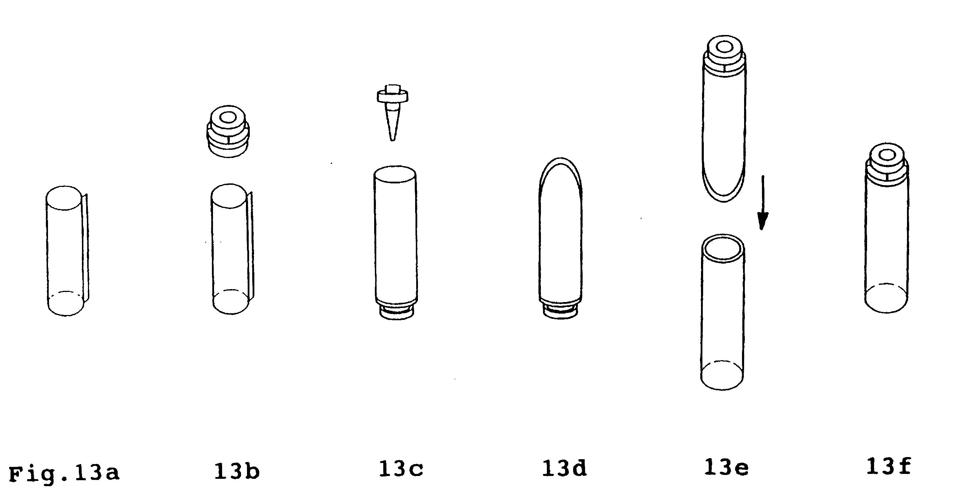

Die Herstellung und Befüllung des erfindungsgemäßen Behälters ist in den Figuren 12 und 13 in Schrägansicht schematisch dargestellt.

Ein gefalteter Folienstreifen wird an den geschnittenen Seiten mit einer Schweißnaht in Längsrichtung versehen, in Abschnitte zerteilt und zu einem Schlauch geformt (Figur 12 a). Das im Spritzgußverfahren hergestellte Unterteil eines zweiteiligen zylindrischen Flansches wird mit dem einen Ende eines derartigen Schlauchabschnittes verschweißt. Das andere Ende des Abschnittes wird mit einer U-förmigen Quernaht verschweißt (Figuren 12 b und 12 c). Der fertige Behälter (Figur 12 c) wird in eine zylindrische Hülse aus Aluminium geschoben (Figur 12 d), deren Rand in eine Rille im Rand des zylindrischen Flansches gedrückt wird. Dadurch ist der Behälter mit der Hülse fest verbunden. Der in der Hülse befindliche leere Behälter wird durch das mit einer Öffnung versehene Unterteil des Flansches mit einem Fluid befüllt (Figur 12 e). Nach dem Befüllen wird das Oberteil des Flansches in das Unterteil eingedrückt (Figur 12 f), und beide Teile werden miteinander dicht verbunden. Das Fertigteil (Figur 12 g) ist zum Aufstecken auf einen Entnahmestutzen bereit.

Ein weiteres Herstellungsverfahren ist in Figur 13 dargestellt. Ein Abschnitt eines mit einer Längsnaht versehenen Folienschlauches (Figur 13 a) wird an seinem einen Ende mit einem einstückigen Flansch, der mit einer Siegelfolie verschlossen ist, verbunden (Figur 13 b). Der Behälter wird durch das offene andere Ende des Folienschlauches mit einem Fluid befüllt (Figur 13 c). Das offene Ende des Folienschlauches wird mit einer U-förmigen Quernaht verschlossen (Figur 13 d). Der gefüllte Behälter wird in eine Hülse aus Kunststoff geschoben (Figur 13 e). Der Rand der Kunststoffhülse wird auf den Rand des formstabilen Flansches aufgeschnappt. Das Fertigteil (Figur 13 f) ist zum Aufstecken auf einen Entnahmestutzen bereit.

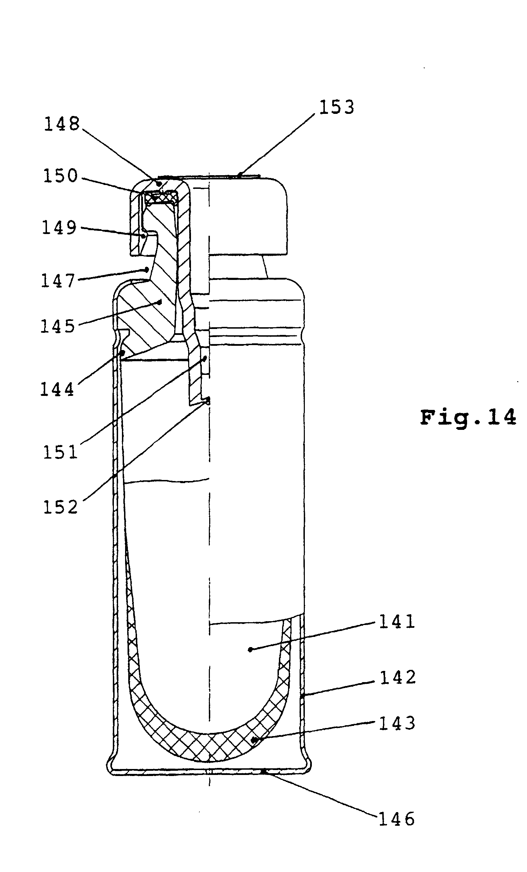

In Figur 14 ist ein typischer mit einem Fluid gefüllter erfindungsgemäßer Behälter (141) teilweise im Querschnitt dargestellt, der sich in einer Metallhülse (142) befindet. Der Folienbeutel ist an seinem einen Ende mit einer U-Naht (143) verschweißt und an seinem anderen Ende mit dem Rand (144) des Unterteils (145) eines zweistückigen zylindrischen formstabilen Flansches verschweißt. Die Metallhülse hat einen Boden (146), in dem sich ein Loch befindet, durch das Luft in den Raum zwischen Metallhülse und Folienbeutel eintreten kann. Das offene Ende der Hülse ist in eine Rille (147) im Rand des Unterteils (145) gedrückt. Die Hülse ist mit dem Flansch fest verbunden. Das Oberteil (148) des Flansches ist in eine Öffnung im Unterteil eingesetzt. Unterteil und Oberteil sind mit einem Schnappverschluß (149) verbunden und mit einer Flachdichtung (150) versehen. Der Führungskanal (151) ist an seinem inneren Ende mit einer Membran (152) verschlossen und an seinem äußeren Ende mit einer siegelfolie (153) versiegelt.

Die in Figur 14 dargestellte Metallhülse besteht aus Aluminium. Sie ist 43 mm lang und hat einen Außendurchmesser von 17 mm und eine 0,5 mm dicke Wand. Der zweistückige Flansch besteht aus Polyethylen und ist im Spritzgußverfahren hergestellt. Das Oberteil des Flansches ist einschließlich Membran in einem Verfahrensschritt gefertigt. Der Führungskanal im Flanschoberteil hat an der Stelle der Preßpassung einen Innendurchmesser von 2,5 mm und paßt stramm auf einen Entnahmestutzen.

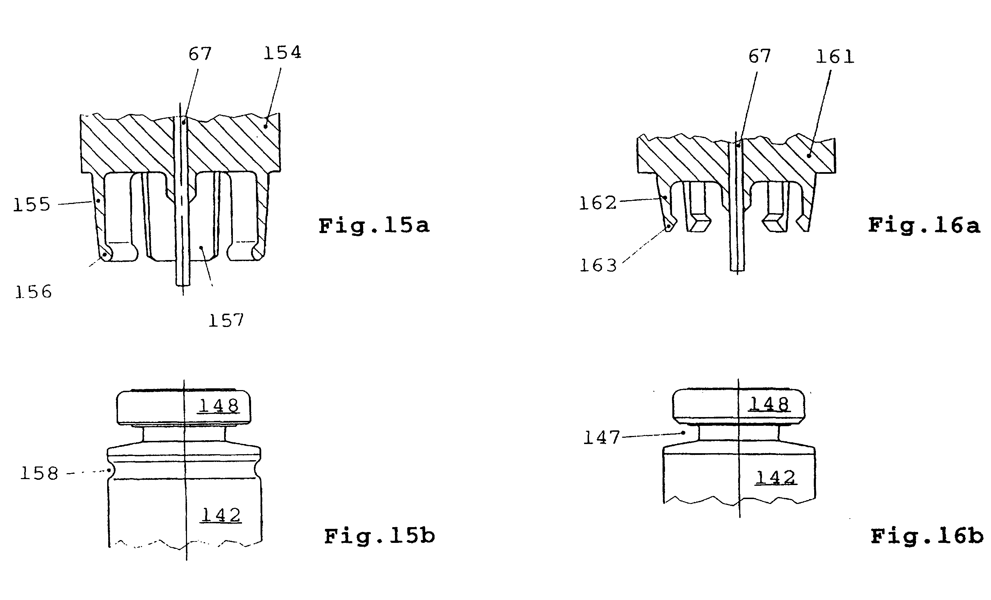

In den Figuren 15 a und 15 b ist eine lösbare formschlüssige steckbare Schnappverbindung zwischen dem formscabilen Flansch des Behälters und dem Anschlußstück in einem Entnahmegerät dargestellt.

Figur 15 a zeigt einen Querschnitt durch das im Entnahmegerät befindliche Anschlußstück (154), das auf seiner Achse den Entnahmestutzen (67) enthält. Der Entnahmestutzen ist umgeben von mehreren Schnapphaken (155) mit Schnappnasen (156) mit rundem Querschnitt. Die Schnapphaken sind durch Zwischenräume voneinander getrennt und können eine azimutale Breite von 10 Grad bis 60 Grad haben. Zwischen zwei Schnapphaken (155) kann ein Abschnitt (157) vorhanden sein, der keine Schnappnase enthält. Dieser Abschnitt legt sich formschlüssig an die Außenwand des eingesteckten Behälters an.

Figur 15 b zeigt in Seitenansicht das Ende eines in einer Hülse (142) befindlichen Behälters, dessen formstabiler Flansch (148) aus der Hülse herausragt. Die Hülse hat - entsprechend Figur 14 - bevorzugt im Bereich des in die Hülse hineinragenden Teils des Flansches eine umlaufende Rille (158), in die die Schnappnasen (156) des in das Anschlußstück (154) eingesteckten Behälters eingreifen, wodurch der Behälter mit dem Anschlußstück lösbar und formschlüssig verbunden wird.

In den Figuren 16 a und 16 b ist eine weitere Ausführungsform einer lösbaren, formschlüssigen, steckbaren Schnappverbindung dargestellt.

Figur 16 a zeigt einen Querschnitt durch das im Entnahmegerät befindliche Anschlußstück (161), das auf seiner Achse den Entnahmestutzen (67) enthält. Der Entnahmestutzen ist umgeben von mehreren zungenförmigen Schnapphaken (162) mit Schnappnasen (163), deren Flanken in beiden Bewegungsrichtungen des Behälters abgeschrägt sind. Die Schnapphaken sind in einem azimutalen Abstand voneinander angebracht.

Figur 16 b zeigt in Seitenansicht das Ende eines in einer Hülse (142) befindlichen Behälters, dessen formstabiler Flansch (148) aus der Hülse herausragt. Die Schnappnasen (163) greifen bei in das Anschlußstück (154) eingestecktem Behälter in die umlaufende Rille (147) des aus der Hülse herausragenden Teils des formstabilen Flansches (148) ein, wodurch der Behälter mit dem Anschlußstück lösbar und formschlüssig verbunden wird.

## Patentansprüche

1. Verwendung eines gas- und flüssigkeitsdichten Behälters als auswechselbare Kartusche für eine medizinische Flüssigkeit in einem treibgasfreien Zerstäuber, welcher eine Entnahmestutzen in Form eines Hohlkolbens aufweist, wobei der Behälter umfasst
- einen Folienbeutel (11, 21, 31), der an beiden Enden verschlossen ist, wobei mindestens ein Ende durch eine Schweißnaht (13, 23, 32) verschlossen ist, die im wesentlichen quer zur Beutelachse verläuft und der Folienbeutel bei einem Differenzdruck zwischen dem Innenraum des Behälters und seiner Umgebung unterhalb 300 hPa (300 mbar) durch den Außendruck verformbar ist,
- einen formstabilen Flansch (15, 25, 34), der an dem Folienbeutel dicht angebracht ist und der als lösbares Verbindungselement zum Aufstecken des Behälters auf einen Entnahmestutzen (67) ausgebildet ist,
- einen Führungskanal (42, 54) in dem Flansch,
- wobei in dem Führungskanal eine Dichtstelle (56, 64, 74) ausgebildet ist und/oder eine Presspassung (55, 66, 77), die den Entnahmestutzen umschließt
- und eine Entnahmestelle für die Flüssigkeit im Bereich des Führungskanals, in die bei Gebrauch der Hohlkolben sticht, so daß er in die medizinische Flüssigkeit eintaucht.

2. Verwendung nach Anspruch 1, wobei der Behälter **gekennzeichnet ist durch**
- einen Folienbeutel, der bei einem Differenzdruck unterhalb 150 hPa (150 mbar), bevorzugt unterhalb 80 hPa (80 mbar) verformbar ist.

3. Verwendung nach den Ansprüchen 1 und 2, wobei der Behälter **gekennzeichnet ist durch**
- einen Folienbeutel (31), der an beiden Enden **durch** eine Schweißnaht (32) verschlossen ist,
- und einen formstabilen Flansch (34), der am Folienbeutel (31) dicht angebracht ist.

4. Verwendung nach den Ansprüchen 1 und 2, wobei der Behälter **gekennzeichnet ist durch**
- einen Folienbeutel (11, 21), der an einem Ende **durch** eine Schweißnaht (13, 23) und am anderen Ende **durch** den formstabilen Flansch (12, 24) dicht verschlossen ist.

5. Verwendung nach den Ansprüchen 1 bis 4, wobei der Behälter **gekennzeichnet ist durch**
- einen formstabilen bevorzugt rotationssymmetrischen Flansch (41, 51, 61), dessen Größe an die Größe des Folienbeutels angepaßt ist,
- und mit dem der Folienbeutel an einem Ende verschlossen ist.

6. Verwendung nach den Ansprüchen 1 bis 5, wobei der Behälter **gekennzeichnet ist durch** einen Führungskanal (42, 54) mit einer Dichtstelle (56, 64, 74).

7. Verwendung nach den Ansprüchen 1 bis 6, wobei der Behälter **gekennzeichnet ist durch** einen formstabilen Flansch mit Preßpassung (55, 66, 77) innerhalb des Führungskanals, der den Entnahmestutzen umschliesst.

8. Verwendung nach den Ansprüchen 1 bis 7, wobei der Behälter **gekennzeichnet ist durch**
- einen formstabilen Flansch, der aus einem thermoplastischen Kunsstoff besteht.

9. Verwendung nach den Ansprüchen 1 bis 8, wobei der Behälter **gekennzeichnet ist durch**
- eine Schweißnaht (91, 101), die U- oder V-förmig ausgebildet ist und im wesentlichen quer zur Beutelachse verläuft, und die sich teilweise in Richtung der Beutelachse erstreckt, oder eine T-förmig ausgebildete Schweißnaht (111).

10. Verwendung nach den Ansprüchen 1 bis 9, wobei der Behälter **gekennzeichnet ist durch**
- eine Dichtstelle in Form eines in einer Nut liegenden Ringes (54, 74) aus elastischem Material.

11. Verwendung nach den Ansprüchen 1 und 10, wobei der Behälter **gekennzeichnet ist durch**
- eine Entnahmestelle, die als Einstichstelle ausgebildet ist.

12. Verwendung nach den Ansprüchen 1 bis 11, wobei der Behälter **gekennzeichnet ist durch**
- eine Entnahmestelle, die mit einer durchstechbaren Membran versehen ist.

13. Verwendung nach den Ansprüchen 1 bis 12, wobei der Behälter **gekennzeichnet ist durch**
- eine Entnahmestelle, die mit einer durchstechbaren Membran versehen ist, wobei
- die Membran (43, 78) am Ende oder innerhalb des Führungskanals angebracht ist,
- oder die Membran (86) ein Teil des Folienbeutels ist.

14. Verwendung nach den Ansprüchen 1 bis 13, wobei der Behälter **gekennzeichnet ist durch**
- eine Entnahmestelle, die mit einer Siegelfolie (58, 84, 151) versiegelt ist.

15. Verwendung nach den Ansprüchen 1 bis 14, wobei der Behälter **gekennzeichnet ist durch**
- einen Folienbeutel (11, 21, 31) aus einer Folie aus Kunststoff, Metall oder Metall-Legierung
- oder einen Folienbeutel aus einer Verbundfolie aus Kunststoff und Metall,
- oder einen Folienbeutel aus einer Kunststoff-Folie mit einer Schicht aus Metall, Glas oder Keramik.

16. Verwendung nach den Ansprüchen 1 bis 15, wobei der Behälter **gekennzeichnet ist durch**
- einen Folienbeutel mit
- einer Innenfolie aus Kunststoff und einer Außenfolie aus Metall
- oder mit zwei Folien aus unterschiedlichen Kunststoffen.

17. Verwendung nach den Ansprüchen 1 bis 15, wobei der Behälter **gekennzeichnet ist durch**
- einen Folienbeutel mit
- einer Innenfolie aus einem Copolymer, bevorzugt einem Polyethylen-Copolymer aus Ethylen-Acrylsäure,
- und einer diffusionsdichten Mittelfolie aus Metall oder Kunststoff oder einer diffusionsdichten Mittelschicht aus Metall, Glas oder Keramik,
- und einer Außenfolie aus Kunststoff, dessen Schmelztemperatur höher ist als die Schmelztemperatur der Innenfolie, bevorzugt aus Polyethylen-terephthalat.

18. Verwendung nach den Ansprüchen 1 bis 17, wobei der Behälter **gekennzeichnet ist durch**
- einen Folienbeutel aus einer Kunststoff-Folie mit einer Dicke von 20 µm bis 100 µm,
- oder einen Folienbeutel aus einer Verbundfolie mit einer Innenfolie aus Kunststoff mit einer Dicke von 20 µm bis 100 µm und einer Außenfolie aus Metall mit einer Dicke von 8 µm bis 20 µm,
- oder einen Folienbeutel aus einer Verbundfolie mit einer Innenfolie aus Kunststoff mit einer Dicke von 20 µm bis 100 µm, einer Mittelfolie aus Metall mit einer Dicke von 8 µm bis 20 µm und einer Außenfolie aus Kunststoff mit einer Dicke von 10 µm bis 40µm.

19. Verwendung nach den Ansprüchen 1 bis 18, wobei der Behälter **gekennzeichnet ist durch**
- ein Füllvolumen von 0,5 Milliliter bis 5 Milliliter, bevorzugt von 1 ml bis 4 ml.

20. Verwendung nach den Ansprüchen 1 bis 19, wobei der Behälter **gekennzeichnet ist durch**
- ein Füllvolumen von 1 ml bis 4 ml oder von 2 ml bis 4 ml.

21. Verwendung nach den Ansprüchen 1 bis 20, **dadurch gekennzeichnet, daß** sich der Behälter
- in einer formstabilen Hülse (142) aus Metall oder aus Kunststoff befindet, die mit dem Flansch verbunden ist.

22. Verwendung nach den Ansprüchen 1 bis 21, **dadurch gekennzeichnet, daß** sich der Behälter in einer formstabilen Hülse mit einem Durchmesser von 10 mm bis 30 mm, bevorzugt von 12 mm bis 17 mm befindet.

23. Verwendung nach den Ansprüchen 1 bis 22, **dadurch gekennzeichnet, daß** sich der Behälter in einer formstabilen Hülse befindet, aus der der formstabile Flansch hervorragt, wobei die Länge über alles von 20 mm bis 60 mm, bevorzugt von 30 mm bis 50 mm beträgt.

24. Verwendung nach den Ansprüchen 21 bis 23, wobei die Hülse **gekennzeichnet ist dadurch**, daß
- sie im wesentlichen rundum geschlossen ist,
- eine Öffnung enthält,
- oder bei der an der Verbindungsstelle mit dem Flansch ein Spalt vorhanden ist,
- oder daß sie als Korb mit vielen Öffnungen ausgebildet ist
- oder daß sie als Bügel ausgebildet ist.

25. Verwendung nach den Ansprüchen 21 bis 24, wobei der Behälter **dadurch gekennzeichnet ist, daß**
- er eine umlaufende Rille (158; 147) im formstabilen Flansch aufweist.

26. Verwendung des Behälters nach den Ansprüchen 1 bis 25
- als Primärverpackung für eine medizinische Flüssigkeit, die in vielen Teilmengen aus dem Folienbeutel entnommen wird.

27. Verwendung des Behälters nach den Ansprüchen 1 bis 26
- als Primärverpackung für eine medizinische Flüssigkeit, die jeweils in einer Dosis von 10 Mikroliter bis 50 Mikroliter, bevorzugt von 15 µl bis 20 µl portionsweise entnommen wird.

28. Verwendung des Behälters nach den Ansprüchen 1 bis 27
- als Kartusche, enthaltend eine inhalierbare Arzneimittel-Zubereitung, die als Lösung in Ethanol oder in Ethanol/Wasser oder in Wasser vorliegt.

29. Verwendung des Behälters nach den Ansprüchen 1 bis 28
- als Kartusche, enthaltend eine inhalierbare Arzneimittel-Zubereitung mit einem Wirkstoff oder mit mehreren Wirkstoffen, wie Berotec (Fenoterol-Hydrobromid;1-(3,5-dihydroxyphenyl)-2-[1-(4-hydroxy-benzyl)-ethyl]-amino]-ethanolhydrobromid), Atrovent (Ipratropiumbromid), Berodual (Kombination aus Fenoterol-Hydrobromid und Ipratropium-bromid), Salbutamol (oder Albuterol), Combivent, Oxivent (Oxitropiumbromid), Ba 679 (Tiotropiumbromid), BEA 2108 (Di- (2-thienyl)-glykolsäuretropenolester), Beclomethason, Flunisolid, Budesonid.

30. Verwendung des Behälters nach den Ansprüchen 1 bis 29
- mit einer Arzneimittel-Zubereitung zur Behandlung von Erkankungen mittels inhalierbarer Wirkstoffe.

31. Verwendung nach einem der Ansprüche 1 bis 30, **dadurch gekennzeichnet, daß**
- der Zerstäuber ein Entnahmegerät ist, das mit einem Anschlußstück versehen ist,
- in das der Behälter durch eine lösbare, formschlüssige, steckbare Schnappverbindung zwischen dem Anschlußstück (154; 161) im Entnahmegerät und dem formstabilen Flansch (148) des Behälters eingesteckt wird.

32. Verwendung nach Anspruch 31, **gekennzeichnet durch**
- eine umlaufende Rille (158; 147) im formstabilen Flansch, in die die am Anschlußstück (154; 161) angebrachten Schnappnasen (156; 163) der Schnapphaken (155; 162) bei in das Anschlußstück eingestecktem Behälter eingreifen.

33. Treibgasfreien Zerstäuber mit einer auswechselbaren Kartusche in Form eines Behälters für eine medizinische Flüssigkeit mit Merkmalen wie in einem der Ansprüche 1 bis 32 definiert, wobei der Inhaltor einen Entnahmestutzen in Form eines Hohlkolbens (67) zur Entnahme der Flüssigkeit aus dem Behälter aufweist.

## Claims

1. Use of a gas-tight and fluid-tight container as a replaceable cartridge for a medicinal fluid in a propellant-free atomiser, which has a discharge connection piece in the form of a hollow piston, wherein the container comprises
- a film bag (11, 21, 31) which is closed at both ends, wherein at least one end is closed by a weld seam (13, 23, 32) which extends substantially transversely to the axis of the bag, and the film bag is deformable by external pressure when there is a pressure differential between the internal space of the container and its surroundings of less than 300 hPa (300 mbar),
- a flange (15, 25, 34) of stable shape which is arranged sealingly on the film bag and which can be designed as a detachable connection element for placing the container on a discharge connection piece (67),
- a guide passage (42, 54) in the flange,
- wherein a sealing location (56, 64, 74) is formed in the guide passage and/or a force fit (55, 66, 77) embracing the discharge connection piece,
- and a discharge location for the fluid in the region of the guide passage into which the hollow piston plunges during use, so that it is immersed in the medicinal fluid.

2. Use according to Claim 1, wherein the container is **characterised by**
- a film bag which is deformable when there is a pressure differential of below 150 hPa (150 mbar), preferably of below 80 hPa (80 mbar).

3. Use according to Claims 1 and 2, wherein the container is **characterised by**
- a film bag (31) which is closed at both ends by a weld seam (32),
- and a flange (34) of stable shape which is applied sealingly to the film bag (31).

4. Use according to Claims 1 and 2, wherein the container is **characterised by**
- a film bag (11, 21) which is sealingly closed at one end by a weld seam (13, 23) and at the other end by the flange (12, 24) of stable shape.

5. Use according to Claims 1 to 4, wherein the container is **characterised by**
- a flange (41, 51, 61) of stable shape which is preferably rotationally-symmetrical and which is adapted in size to the size of the film bag,
- and by means of which the film bag is closed at one end.

6. Use according to Claims 1 to 5, wherein the container is **characterised by** a guide passage (42, 54) with a sealing location (56, 64, 74).

7. Use according to Claims 1 to 6, wherein the container is **characterised by** a flange of stable shape with a force fit (55, 66, 77) inside the guide passage which embraces the discharge connection piece.

8. Use according to Claims 1 to 7, wherein the container is **characterised by**
- a flange of stable shape which consists of a thermoplastics plastics material.

9. Use according to Claims 1 to 8, wherein the container is **characterised by**
- a weld seam (91, 101) which is of a U-shaped or V-shaped configuration and which extends substantially transversely to the axis of the bag, and which extends partly in the direction of the axis of the bag, or a T-shaped weld seam (111).

10. Use according to Claims 1 to 9, wherein the container is **characterised by**
- a sealing location in the form of a ring (54, 75) of elastic material which is disposed in a groove.

11. Use according to Claims 1 and 10, wherein the container is **characterised by**
- a discharge location which is in the form of a push-up location.

12. Use according to Claims 1 to 11, wherein the container is **characterised by**
- a discharge location which is provided with a pierceable membrane.

13. Use according to Claims 1 to 12, wherein the container is **characterised by**
- a discharge location which is provided with a pierceable membrane, wherein
- the membrane (48, 78) is arranged at the end of, or inside, the guide passage,
- or the membrane (86) is a part of the film bag.

14. Use according to Claims 1 to 13, wherein the container is **characterised by**
- a discharge location which is sealed with a sealing film (58, 84, 151).

15. Use according to Claims 1 to 14, wherein the container is **characterised by**
- a film bag (11, 21, 31) consisting of a plastic, metal or metal-alloy film
- or a film bag consisting of a composite film of plastics material and metal,
- or a film bag consisting of a plastic film with a metal, glass or ceramic layer.

16. Use according to Claims 1 to 15, wherein the container is **characterised by**
- a film bag with
- an inner film of plastics material and an outer film of metal
- or with two films of different plastics materials.

17. Use according to Claims 1 to 15, wherein the container is **characterised by**
- a film bag with
- an inner film consisting of a copolymer, preferably a polyethylene copolymer of ethylene-acrylic acid,
- and a diffusion-tight central film consisting of metal or plastics material, or a diffusion-tight central layer consisting of metal, glass or ceramics material,
- and an outer film of plastics material, the melting temperature of which is higher than the melting temperature of the inner film, which is preferably polyethylene-terephthalate.

18. Use according to Claims 1 to 17, wherein the container is **characterised by**
- a film bag consisting of a plastic film of between 20 :m and 100 :m in thickness,
- or a film bag consisting of a composite film with an inner film of plastics material of between 20 :m and 100 :m in thickness, and an outer film of metal of between 8 :m and 20 :m in thickness,
- or a film bag consisting of a composite film with an inner film of plastics material of between 20 :m and 100 :m in thickness, a central film of metal of between 8 :m and 20 :m in thickness, and an outer film of plastics material of between 10 :m and 40 :m in thickness.

19. Use according to Claims 1 to 18, wherein the container is **characterised by**
- a filling capacity of between 0.5 millilitres and 5 millilitres, preferably of between 1 ml and 4 ml.

20. Use according to Claims 1 to 19, wherein the container is **characterised by**
- a filling capacity of between 1 ml and 4 ml, or of between 2 ml and 4 ml.

21. Use according to Claims 1 to 20, **characterised in that** the container is disposed in a metal or plastic sleeve (142) of stable shape which is connected to the flange.

22. Use according to Claims 1 to 21, **characterised in that** the container is disposed in a sleeve of stable shape which is between 10 mm and 30 mm, preferably between 12 mm and 17 mm, in diameter.

23. Use according to Claims 1 to 22, **characterised in that** the container is disposed in a sleeve of stable shape from which the flange of stable shape projects, wherein the overall length is between 20 mm and 60 mm, preferably between 30 mm and 50 mm.

24. Use according to Claims 21 to 23, wherein the sleeve is **characterised in that**
- it is closed substantially all around,
- it has an opening,
- or wherein there is a gap at the connection location to the flange,
- or that it is designed as a basket with many openings
- or that it is in the form of a stirrup member.

25. Use according to Claims 21 to 24, wherein the container is **characterised in that**
- it has a peripheral groove (158; 147) in the flange of stable shape.

26. Use of the container according to Claims 1 to 25
- as primary packaging for a medicinal fluid which is discharged from the film bag in a plurality of partial quantities.

27. Use of the container according to Claims 1 to 26
- as a primary packaging for a medicinal fluid which is discharged in portion-wise manner in doses of between 10 microlitres and 50 microlitres, preferably of between 15 :1 and 20 :1.

28. Use of the container according to Claims 1 to 27
- as a cartridge containing an inhalant medicinal preparation which is present as a solution in ethanol or in ethanol/water or in water.

29. Use of the container according to Claims 1 to 28
- as a cartridge containing an inhalant medicinal preparation with one active substance, or with a plurality of active substances, such as Berotec (Fenoterolhydrobromide; 1-(3,5-dihydroxyphenyl)-2-[1-(4-hydroxy-benzyl)-ethyl]-amino] - ethanolhydrobromide), Atrovent (Ipratropium bromide), Berodual (combination of feneterol-hydrobromide and Ipratroprium-bromide), Salbutamol (or Albuterol), Combivent, Oxivent (Oxitropium bromide), Ba 679 (Tiotropium bromide), BEA 2108 (Di- (2-thienyl)-glycolic acid tropenol ester), Beclomethason, Flunisolid, Budesonid.

30. Use of the container according to Claims 1 to 29
- with a medicinal preparation for the treatment of illnesses using inhalant substances.

31. Use according to one of Claims 1 to 30, **characterised in that**
- the atomiser is a discharge instrument which is provided with a connection piece,
- into which the container is inserted by means of a releasable, positive, plug-in snap connection between the connection piece (154; 161) in the discharge instrument and the flange (148) of stable shape of the container.

32. Use according to Claim 31, **characterised by**
- a peripheral groove (158; 147) in the flange of stable shape, into which groove the snap connection lugs (156; 163) of the snap connection hooks (155; 162) arranged on the connection piece (154; 161) engage when the container is inserted into the connection piece.

33. A propellant-free atomiser with a replaceable cartridge in the form of a container for a medicinal fluid having features such as defined in one of Claims 1 to 32, wherein the inhaler has a discharge connection piece in the form of a hollow piston (67) for discharging the fluid from the container.

## Revendications

1. Utilisation d'un récipient étanche au gaz et au liquide comme cartouche échangeable pour un liquide médical dans un diffuseur sans gaz propulseur, lequel comprend un support de prélèvement sous forme d'un piston creux, dans laquelle le récipient comprend
- un sac en film (11, 21, 31), qui est fermé aux deux extrémités, dans laquelle au moins une extrémité est fermée par une soudure (13, 23, 32), qui s'étend sensiblement transversalement par rapport à l'axe du sac et le sac en film peut être déformé par la pression extérieure lors d'une différence de pression, entre l'espace intérieur du récipient et son environnement, située au-dessous de 300 hPa (300 mbar),
- une bride indéformable (15, 25, 34), qui est aménagée de façon étanche sur le sac en film et qui est conçue comme un élément de raccordement détachable pour fixer le récipient à un support de prélèvement (67),
- un canal de guidage (42, 54) dans la bride,
- dans laquelle une zone d'étanchéité (56, 64, 74) est conçue dans le canal de guidage et/ou un ajustage serré (55, 66, 77), qui entoure le support de prélèvement,
- et un point de prélèvement pour le liquide dans la zone du canal de guidage, dans lequel perce le piston creux lors d'une utilisation, de sorte que celui-ci plonge dans le liquide médical.

2. Utilisation selon la revendication 1, dans laquelle le récipient est **caractérisé par**
- un sac en film qui peut être déformé lors d'une différence de pression inférieure à 150 hPa (150 mbar), de préférence inférieure à 80 hPa (80 mbar).

3. Utilisation selon les revendications 1 et 2, dans laquelle le récipient est **caractérisé par**
- un sac en film (31) qui est fermé au deux extrémités par une soudure (32),
- et une bride indéformable (34) qui est aménagée de manière étanche sur le sac en film (31).

4. Utilisation selon les revendications 1 et 2, dans laquelle le récipient est **caractérisé par**
- un sac en film (11, 21), qui est fermé de manière étanche à une extrémité par une soudure (13, 23) et à l'autre extrémité par la bride (12, 24) indéformable.

5. Utilisation selon les revendications 1 à 4, dans laquelle le récipient est **caractérisé par**
- une bride (41, 51, 61) indéformable de préférence à symétrie de révolution, dont la taille est adaptée à la taille du sac en film,
- et avec laquelle le sac en film est fermé à une extrémité.

6. Utilisation selon les revendications 1 à 5, dans laquelle le récipient est **caractérisé par** un canal de guidage (42, 54) comportant une zone d'étanchéité (56, 64, 74).

7. Utilisation selon les revendications 1 à 6, dans laquelle le récipient est **caractérisé par** une bride indéformable comprenant un ajustage serré (55, 66, 77) à l'intérieur du canal de guidage, qui entoure le support de prélèvement.

8. Utilisation selon les revendications 1 à 7, dans laquelle le récipient est **caractérisé par**
- une bride indéformable qui se compose d'une matière plastique thermoplastique.

9. Utilisation selon les revendications 1 à 8, dans laquelle le récipient est **caractérisé par**
- une soudure (91, 101), en forme de U ou de V, et qui s'étend sensiblement transversalement par rapport à l'axe du sac, et qui s'étend partiellement en direction de l'axe du sac, ou une soudure (111) en forme de T.

10. Utilisation selon les revendications 1 à 9, dans laquelle le récipient est **caractérisé par**
- une zone d'étanchéité sous forme d'un anneau (54, 74) situé dans une rainure et fabriqué dans un matériau élastique.

11. Utilisation selon les revendications 1 et 10, dans laquelle le récipient est **caractérisé par**
- un point de prélèvement qui est conçu comme point d'encoche.

12. Utilisation selon les revendications 1 à 11, dans laquelle le récipient est **caractérisé par**
- un point de prélèvement qui est doté d'une membrane pouvant être transpercée.

13. Utilisation selon les revendications 1 à 12, dans laquelle le récipient est **caractérisé par**
- un point de prélèvement qui est doté d'une membrane pouvant être transpercée, dans lequel
- la membrane (43, 78) est aménagée sur l'extrémité ou à l'intérieur du canal de guidage,
- ou la membrane (86) est une partie du sac en film.

14. Utilisation selon les revendications 1 à 13, dans laquelle le récipient est **caractérisé par**
- un point de prélèvement qui est scellé par un film de scellement (58, 84, 151).

15. Utilisation selon les revendications 1 à 14, dans laquelle le récipient est **caractérisé par**
- un sac en film (11, 21, 31) fabriqué dans un film en matière plastique, en métal ou en un alliage en métal,
- ou un sac en film fabriqué dans un film composé de matière plastique et de métal,
- ou un sac en film fabriqué dans un film en matière plastique et comprenant une couche de métal, de verre ou de céramique.

16. Utilisation selon les revendications 1 à 15, dans laquelle le récipient est **caractérisé par**
- un sac en film avec
- un film intérieur en plastique et un film extérieur en métal
- ou avec deux films fabriqués dans différentes matières plastiques.

17. Utilisation selon les revendications 1 à 15 , dans laquelle le récipient est **caractérisé par**
- un sac en film avec
- un film intérieur fabriqué dans un copolymère, de préférence dans un copolymère de polyéthylène d'éthylène-acide acrylique,
- et un film central étanche à la diffusion fabriqué dans un métal ou une matière plastique ou une couche centrale étanche à la diffusion en métal, en verre ou en céramique,
- et un film extérieur en matière plastique, dont la température de fusion est supérieure à la température de fusion du film intérieur, de préférence en polyéthylène-téréphtalate.

18. Utilisation selon les revendications 1 à 17, dans laquelle le récipient est **caractérisé par**
- un sac en film fabriqué dans un film en matière plastique dont l'épaisseur est comprise entre 20 µm et 100 µm,
- ou un sac en film fabriqué dans un film composé d'un film intérieur en matière plastique, dont l'épaisseur est comprise entre 20 µm et 100 µm, et un film extérieur en métal dont l'épaisseur est comprise entre 8 µm et 20 µm,
- ou un sac en film fabriqué dans un film composé d'un film intérieur en matière plastique, dont l'épaisseur est comprise entre 20 µm et 100 µm, un film central en métal dont l'épaisseur est comprise entre 8 µm et 20 µm, et un film extérieur en matière plastique dont l'épaisseur est comprise entre 10 µm et 40 µm.

19. Utilisation selon les revendications 1 à 18, dans laquelle le récipient est **caractérisé par**
- un volume de remplissage compris entre 0,5 millilitres et 5 millilitres, de préférence entre 1 ml et 4 ml.

20. Utilisation selon les revendications 1 à 19, dans laquelle le récipient est **caractérisé par**
- un volume de remplissage compris entre 1 ml et 4 ml, ou 2 ml et 4 ml.

21. Utilisation selon les revendications 1 à 20, **caractérisée en ce que** le récipient
- se situe dans une enveloppe (142) indéformable en métal ou en matière plastique, qui est reliée à la bride.

22. Utilisation selon les revendications 1 à 21, **caractérisée en ce que** le récipient se situe dans une enveloppe indéformable dont le diamètre mesure de 10 mm à 30 mm, de préférence de 12 mm à 17 mm.

23. Utilisation selon les revendications 1 à 22, **caractérisée en ce que** le récipient se situe dans une enveloppe indéformable, fait saillie de la bride indéformable, dans laquelle la longueur totale mesure de 20 mm à 60 mm, de préférence de 30 mm à 50 mm.

24. Utilisation selon les revendications 21 à 23, dans laquelle l'enveloppe est **caractérisée en ce que**
- elle est fermée sensiblement tout autour,
- elle comprend un orifice,
- ou dans laquelle un espace est présent sur le point de raccordement avec la bride,
- ou **en ce qu'**elle est conçue comme un panier comprenant de nombreux orifices
- ou **en ce qu'**elle est conçue comme une pièce courbe.

25. Utilisation selon les revendications 21 à 24, dans laquelle le récipient est **caractérisé en ce que**
- il comprend une cannelure circulaire (158 ; 147) dans la bride indéformable.

26. Utilisation du récipient selon les revendications 1 à 25
- comme emballage primaire pour un liquide médical, qui est prélevé du sac en film en plusieurs parties.

27. Utilisation du récipient selon les revendications 1 à 26
- comme emballage primaire pour un liquide médical, qui est prélevé respectivement dans une dose comprise entre 10 microlitres et 50 microlitres, de préférence entre 15 µl et 20 µl par portions.

28. Utilisation du récipient selon les revendications 1 à 27
- comme cartouche, contenant une préparation médicamenteuse pouvant être inhalée, qui se présente comme une solution dans l'éthanol ou dans l'éthanol/eau ou dans l'eau.

29. Utilisation du récipient selon les revendications 1 à 28
- comme cartouche, contenant une préparation médicamenteuse pouvant être inhalée avec une substance active ou plusieurs substances actives, comme Berotec (fenoterol bromhydrate ;I-(3,5-dihydroxyphényl)-2-[1-(4-hydroxy-benzyl)-éthyl]-amino]-éthanolbromhydrate), Atrovent (Ipratropium bromure), Berodual (combinaison de fenoterol bromhydrate et ipratropium bromure), Salbutamol (ou l'Albuterol), Combivent, Oxivent (Oxitropium bromure), Ba679 (bromure de tiotropium), BEA 2108 (Di-(2-thienyl)-acide glycolique tropenolester), Beclomethason, Flunisolid, Budesonid.

30. Utilisation du récipient selon les revendications 1 à 29
- avec une préparation médicamenteuse pour traiter des maladies au moyen de substances actives pouvant être inhalées.

31. Utilisation selon l'une quelconque des revendications 1 à 30, **caractérisée en ce que**
- le diffuseur est un appareil de prélèvement, qui est doté d'une pièce de raccord,
- dans lequel le récipient est inséré par l'intermédiaire d'un assemblage à encliquetage pouvant être introduit, par concordance des formes et détachable entre la pièce de raccord (154 ; 161) dans l'appareil de prélèvement et la bride (148) indéformable du récipient.

32. Utilisation selon la revendication 31, **caractérisée par**
- une cannelure circulaire (158 ; 147) dans la bride indéformable, dans laquelle les becs à déclic (156 ; 163) des crochets à déclic (155 ; 162), aménagés sur la pièce de raccord (154 ; 161), engagent dans le cas du récipient introduit dans la pièce de raccord.

33. Diffuseur sans gaz propulseur avec une cartouche échangeable sous forme d'un récipient pour un liquide médical avec les caractéristiques comme définies dans l'une quelconque des revendications 1 à 32, dans laquelle l'inhalateur comprend un support de prélèvement sous forme d'un piston creux (67) pour prélever le liquide du récipient.
